# EUROPÄISCHE PATENTSCHRIFT

(11) **EP 3 721 945 B1**
(45) Veröffentlichungstag und Bekanntmachung des Hinweises auf die Patenterteilung: **14.06.2023**
(21) Anmeldenummer: 20160528.4
(22) Anmeldetag: 03.03.2020
(51) Int. Cl.: A61Q 19/10, A61K 8/37, A61K 8/39, A61K 8/41, A61K 8/44, A61K 8/46, A61K 8/60

(54) **KOSMETISCHE REINIGUNGSZUBEREITUNG**
COSMETIC CLEANSING COMPOSITION
COMPOSITION COSMÉTIQUE DE NETTOYAGE

(30) Priorität: 12.04.2019 DE 102019205293
(43) Veröffentlichungstag der Anmeldung: 14.10.2020
(73) Patentinhaber: Beiersdorf AG, 20253 Hamburg (DE)
(72) Erfinder: Gritza, Denise, 22529 Hamburg (DE); Wilken, Maren, 22848 Norderstedt (DE)
(74) Vertreter: Beiersdorf AG

(56) Entgegenhaltungen:
- DE-A1-102011 084 888
- DATABASE GNPD [Online] MINTEL; 19. Juli 2006 (2006-07-19), anonymous: "Hand & Face Cleaning Gel for Children", XP055715783, gefunden im www.gnpd.com Database accession no. 559180
- DATABASE GNPD [Online] MINTEL; 8. Februar 2008 (2008-02-08), anonymous: "Cream Soap for Hands & Face", XP055715786, gefunden im www.gnpd.com Database accession no. 859877
- DATABASE GNPD [Online] MINTEL; 25. September 2019 (2019-09-25), anonymous: "Enchanting Berry Fragrance 3in1 Shower Gel, Shampoo & Conditioner", XP055715788, gefunden im www.gnpd.com Database accession no. 6895271

## Beschreibung

Kosmetische Produkte dienen im Allgemeinen nicht nur dazu schön und attraktiv auszusehen, sondern sie tragen mit ihrer Wirkung entscheidend zu einem gesteigerten Selbstwertgefühl und zum Wohlbefinden der Menschen bei. Dementsprechend werden die verschiedensten kosmetischen Produkte zur täglichen Reinigung und Pflege der menschlichen Haut eingesetzt.

Die tägliche Körperreinigung mit Duschgelen dient vornehmlich dazu Schmutz, Schweißreste, Fette, abgelagerte Schmutzpartikel und abgestorbene Hautreste effektiv von der Haut zu entfernen. Duschgele enthalten in der Regel anionische Tenside, welche eine besonders effektive Reinigung ermöglichen. Weiterhin enthalten derartige Reinigungsprodukte oftmals zusätzlich weitere Tenside gewählt aus der Gruppe der nichtionischen und amphoteren Tenside.

Bei der Entwicklung von kosmetischen Reinigungszubereitung zur Verwendung als Duschgel konzentrieren sich Kosmetikunternehmen zunächst einmal auf die Personengruppe, die das Produkt nutzen soll und deren spezifische Anforderungen.

Die Reinigungszubereitungen der vorliegenden Erfindung sind als Duschgele speziell für Kinder und ggf. Babys konzeptioniert. Folglich ist es notwendig, dass solche Reinigungszubereitungen auf die spezielle Kinderhaut abgestimmt sind, indem milde Tensidsysteme eingesetzt werden, welche dennoch beim Aufschäumen ein großes Schaumvolumen ausbilden können. Folglich ist als Tensidkomponente Sodium Laureth Sulfate zu vermeiden, da dieses insbesondere bei empfindlicher Haut ein höheres Reizpotential aufweist.

Handelsüblichen Duschgelen werden oftmals Polymere zugesetzt, welche eine flüssige Phase verdicken und/oder ein gummiartiges Gel ausbilden können. Besonders beliebt ist der Einsatz von Acrylat-basierten Polymeren, welche aus Homo- oder Copolymerisation mit Acryl- und/oder Methacrylsäure erhalten werden. Problematisch ist jedoch der Umstand, dass der Einsatz dieser Acrylat-basierten Polymere oder auch Polymere, welche unter der Bezeichnung Polyquaternium bekannt sind, zunehmend in der Kritik steht, da deren biologische Abbaubarkeit nicht vollständig geklärt ist. Somit besteht eine weitere Anforderung darin, Reinigungszubereitungen als Duschgele für Kinder bereitzustellen, welche keine Acrylat-basierten und/oder Polyquaternium Polymere enthalten. Dennoch sollten die Duschgele, die gleiche Textur aufweisen, wie bereits bekannte Handelsübliche Produkte.

Weiterhin ist gerade für Kinder die Sensorik von Duschgelen von entscheidender Bedeutung. Ohne optische Effekte durch Zusatz von Farbstoffen, farbigen Partikeln oder opaleszierenden Substanzen wirken die Zusammensetzungen für Kinder oftmals langweilig, was vermieden werden sollte.

Zusammenfassend ergibt sich somit ein Bedarf an milden Reinigungszubereitungen für Kinder, welche besonders milde zur Haut sein sollen, keine Acrylat-basierten Polymere zur Texturbildung/Verdickung benötigen und gleichzeitig optische ansprechend sind.

Versucht der Fachmann durch Formulierungsversuche diese Vorgabe umzusetzen treten jedoch Probleme auf, die nicht durch allgemeines Fachwissen gelöst werden können.

Ein Problem, welches auftritt ist, dass sich bei Verzicht auf die genannten Komponenten Perlglanzpartikel oftmals nicht über einen längeren Zeitraum stabilisieren lassen. In diesem Zusammenhang heißt stabilisieren, dass ein Ablagern bzw. Aufkonzentrieren von Perlglanzpartikeln in Teilen der Zubereitung vermieden wird. Perlglanzpartikel gemäß der vorliegenden Offenbarung sind die unter den INCI Namen bekannten Substanzen PEG-3 Distearate und Glycol Distearate.

Zubereitungen mit Perlglanzpartikeln sind unter anderem aus DE102010055764 A1 oder DE 10351109 A1 bekannt. Jedoch entsprechend die offenbarten Zubereitungen nicht den Anforderungen und konnten zudem keinen Hinweis auf die vorliegende Erfindung liefern.

### Weiterhin sind dem Fachmann die Produkte Nivea Baby Toddies mit der GNPD Mintel Eintragungsnummern 859877 und 559180 bekannt. Auch diese Produkte konnten keinen Hinweis auf die Erfindung liefern.

Überraschend für den Fachmann wurde nun gefunden, dass das entstehende Problem der Aufkonzentierung von Perlglanzpartikeln in Teilen der Zubereitung vermieden werden kann, durch die vorliegende Erfindung gelöst wird.

Gegenstand der vorliegenden Erfindung ist eine kosmetische Reinigungszubereitung umfassend
a) Sodium Myreth Sulfate;
b) mindestens ein amphoteres Tensid enthaltend eine Betainegruppe;
c) Denatonium Benzoate; und
d) mindestens einen Perlglanzpartikel gewählt aus der Gruppe PEG-3 Distearate und Glycol Distearate
dadurch gekennzeichnet, dass die kosmetische Reinigungszubereitung
- kein Natriumlaurylethersulfat (Sodium Laureth Sulfate) enthält und
- keine Polymere enthalten sind, welche aus Homo- oder Copolymerisation mit Acrylsäure und/oder Methacrylsäure erhalten werden, und/oder keine Polymere enthalten sind, welche gemäß INCI-Bezeichnung mit Polvquaternium-Z bezeichnet werden, wobei Z eine ganzzahlige Zahl im Bereich von 1 bis 100 ist.

Ein weiterer Gegenstand der Erfindung ist die Verwendung einer Kombination aus Sodium Myreth Sulfate, mindestens ein amphoteres Tensid enthaltend eine Betainegruppe und Denatonium Benzoate in einer kosmetischen Reinigungszubereitung enthaltend mindestens einen Perlglanzpartikel gewählt aus der Gruppe PEG-3 Distearate und Glycol Distearate zur Stabilisierung der Perlglanzpartikel in der Reinigungszubereitung, wobei die kosmetische Reinigungszubereitung kein Natriumlaurylethersulfat (Sodium Laureth Sulfate) enthält.

Überraschend zeigte die Kombination der Erfindung, bzw. die erfindungsgemäße Reinigungszubereitung bei Lagerung unter 40°C nach 2 Monaten keine Ablagerungen bzw. Aufkonzentrierungen von Perlglanzpartikeln in der Zubereitung.

Sollten nachfolgend Gewichtsprozentangaben (Gew.-%) ohne Bezugnahme auf eine bestimmte Zusammensetzung oder spezifische Mischung angegeben werden, so beziehen sich diese Angaben immer auf das Gesamtgewicht der kosmetischen Reinigungszubereitung. Sollten nachfolgend Verhältnisse von Komponenten/Substanzen/Stoffgruppen offenbart werden, so beziehen sich diese Verhältnisse auf Gewichtsverhältnisse der genannten Komponenten/ Substanzen/Stoffgruppen.

Die Formulierungen "erfindungsgemäß", "erfindungsgemäß vorteilhaft", "vorteilhaft im Sinne der Vorliegenden Erfindung" etc. beziehen sich im Rahmen der vorliegenden Offenbarung immer sowohl auf die erfindungsgemäße Zubereitung sowie die erfindungsgemäße Verwendung.

Sofern nicht anders angegeben wurden alle Versuche unter Normalbedingungen durchgeführt. Der Begriff "Normalbedingungen" bedeutet 20°C, 1013 hPa und eine relative Luftfeuchtigkeit von 50%.

Wird der Begriff Haut verwendet, so bezieht dieser sich bevorzugt auf die menschliche Haut.

Werden in dieser Offenbarung Viskositätswerte angegeben, so beziehen sich alle Werte auf eine Messung bei 25°C in einer 150 ml Weithalsflasche (VWR Nr.: 807-001) mittels Rheomat R 123 von der Firma proRheo. Der Rheomat R 123 der Firma proRheo GmbH ist ein Rotationsviskosimeter, d. h. ein Messkörper rotiert in der zu vermessenden Substanz. Es wird die Kraft gemessen, die benötigt wird, um den Messkörper in der Probe mit einer vorgegebenen Drehzahl rotieren zu lassen. Aus diesem Drehmoment, der Drehzahl des Messkörpers und den geometrischen Abmessungen des verwendeten Messsystems wird die Viskosität berechnet. Als Messkörper wird der Messkörper Nr.1 (Artikelnr. 200 0191), geeignet für einen Viskositätsbereich bis 10.000 [mPa·s], Drehzahl Bereich 62,5 min⁻¹, verwendet. Erfolgt keine andere Angabe, so erfolgt die Messung zur Viskosität immer 24h nach Herstellung der Zubereitung.

Die erfindungsgemäße kosmetische Reinigungszubereitung ist dadurch gekennzeichnet, dass diese Sodium Myreth Sulfate enthält. Der Anteil von Sodium Myreth Sulfate beträgt vorteilhaft von 4 bis 8 Gew.-%, bevorzugt von 4,2 bis 6 Gew.-% und insbesondere bevorzugt von 4,5 bis 5,5 Gew.-%, jeweils bezogen auf das Gesamtgewicht der Reinigungszubereitung.

Weiterhin ist es vorteilhaft, wenn der Gesamtanteil aller enthaltenen anionischen Tenside kleiner als 8,5 Gew.-%, bevorzugt kleiner 6,5 Gew.-% und insbesondere bevorzugt kleiner 5,6 Gew.-% beträgt, wobei sich die Angaben auf das Gesamtgewicht der Reinigungszubereitung beziehen. Am bevorzugtesten ist es jedoch, wenn neben Sodium Myreth Sulfate keine weiteren anionischen Tenside in der kosmetischen Reinigungszubereitung enthalten sind.

Das oder die erfindungsgemäßen amphoteren Tenside enthaltend eine Betainstruktur sind vorteilhaft gewählt aus der Gruppe Alkylamidobetaine; Alkylbetaine, C_{12/14}Alkyldimethylbetaine; Cocoamidopropylbetaine; Tallow-bis(hydroxyethyl)betaine; Hexadecyldimethylbetaine; Cocodimethylbetaine; Alkylamidopropylsulfobetaine; Alkyldimethylaminbetaine; Cocoamidopropyldimethylbetaine; Alkylamidopropyldimethylaminbetaine; Laurylbetaine; Laurylamidoproplbetaine, Cocoamidobetaine, Laurylamidobetaine, Alkylaminobetaine; Alkylamidobetaine; Cocobetaine; Laurylbetaine; Oleylbetaine; N-alkyldimethylbetaine; C₈-amidobetaine; C₁₂amidobetaine; Lauryldimethylbetaine; Alkylamidepropylbetaine; Amidobetaine; Cetylbetaine; Oleamidopropylbetaine; Isostearamidopropylbetaine; Lauramidopropylbetaine; Cocodimethylbetaine; Isostearamidopropylbetaine; Myristamidopropylbetaine; Palmitamidopropylbetaine; Undecylenamidopropylbetaine und Cocoamidosulfobetaine.

Unter den vorstehend genannten erfindungsgemäßen amphoteren Tenside enthaltend eine Betainstruktur sind insbesondere Cocamidopropylbetaine und/oder Lauramidopropylbetaine bevorzugt.

Der Gesamtanteil der erfindungsgemäßen amphoteren Tenside enthaltend eine Betainstruktur beträgt vorteilhaft von 3 bis 8 Gew.-%, bevorzugt von 4 bis 7 Gew.-% und insbesondere bevorzugt von 4,5 bis 6,5 Gew.-% bezogen auf das Gesamtgewicht der kosmetischen Reinigungszubereitung.

Es ist ebenfalls vorteilhaft, wenn als amphotere Tenside enthaltend eine Betainstruktur Cocamidopropylbetaine und/oder Lauramidopropylbetaine eingesetzt werden und der Gesamtanteil von Cocamidopropylbetaine und/oder Lauramidopropylbetaine von 3 bis 8 Gew.-%, bevorzugt von 4 bis 7 Gew.-% und insbesondere bevorzugt von 4,5 bis 6,5 Gew.-% bezogen auf das Gesamtgewicht der kosmetischen Reinigungszubereitung, beträgt.

Weiterhin ist es vorteilhaft, wenn neben Cocamidopropylbetaine und/oder Lauramidopropylbetaine keine weiteren amphoteren Tenside enthalten sind.

Weiterhin ist es vorteilhaft, wenn das Gewichtsverhältnis von den anionischen Tensiden zu amphoteren Tensiden in der Reinigungszubereitung von 1,5:1 bis 1:1,5, bevorzugt von 1,2:1 bis 1:1,2 und insbesondere bevorzugt von 1,1:1 bis 1:1,1 beträgt.

Weiterhin ist es ebenfalls vorteilhaft im Sinne der vorliegenden Erfindung, wenn der Anteil von Tensiden, welche eine anionische und/oder kationische Ladung aufweisen, weniger als 12 Gew.-%, bevorzugt weniger als 11 Gew.-% und insbesondere bevorzugt weniger als 10,5 Gew.-% bezogen auf das Gesamtgewicht der Zubereitung beträgt.

Die erfindungsgemäße Zubereitung ist weiterhin vorteilhaft dadurch gekennzeichnet, dass diese Denatonium Benzoate in einem Anteil von 0,001 bis 0,01 Gew.-%, bevorzugt 0,002 bis 0,008 Gew.-% und insbesondere bevorzugt von 0,0025 bis 0,005 Gew.-% enthält, wobei sich die Angaben auf das Gesamtgewicht der Zubereitung beziehen.

Ferner enthält die erfindungsgemäße kosmetische Reinigungszubereitung mindestens ein Perlglanzpartikel gewählt aus der Gruppe PEG-3 Distearate und Glycol Distearate.

Der Anteil der Perlglanzpartikel gewählt aus der Gruppe PEG-3 Distearate und Glycol Distearate beträgt vorteilhaft von 0,1 bis 1,5 Gew.-%, bevorzugt 0,2 bis 1 Gew.-% und insbesondere bevorzugt 0,3 bis 0,8 Gew.-%, jeweils bezogen auf das Gesamtgewicht der kosmetischen Reinigungszubereitung.

Es hat sich darüber hinaus gezeigt, dass es vorteilhaft ist, wenn die kosmetische Reinigungszubereitung dadurch gekennzeichnet ist, dass diese zusätzlich PEG-90 Glyceryl Isostearate und/oder PEG-200 Hydrogenated Glyceryl Palmate enthält, wobei es weiterhin vorteilhaft ist, wenn der Gesamtanteil dieser Substanzen von 0,1 bis 4 Gew.-%, bevorzugt 0,3 bis 2 Gew.-% und insbesondere bevorzugt von 0,4 bis 1,5 Gew.-% enthalten sind, jeweils bezogen auf das Gesamtgewicht der kosmetischen Reinigungszubereitung.

Es ist bevorzugt, wenn sowohl PEG-90 Glyceryl Isostearate als auch PEG-200 Hydrogenated Glyceryl Palmate enthalten sind und gleichzeitig der Gewichtsanteil von PEG-90 Glyceryl Isostearate zu PEG-200 Hydrogenated Glyceryl Palmate von 1,4:1 bis 1:1,4, insbesondere bevorzugt von 1,2:1 bis 1:1,2 beträgt.

Erfindungsgemäß vorteilhaft umfasst die kosmetische Reinigungszubereitung mindestens ein Alkyl(oligo)glycosid der allgemeinen Formel RO-[G]ₓ, in der R für einen Alkyl- und/oder Alkylenrest mit 4 bis 22 Kohlenstoffatomen, G für einen Zuckerrest mit 5 oder 6 Kohlenstoffatomen und X für eine ganzzahlige Zahl im Bereich von 1 bis 10 steht. Bevorzugt sind die unter den INCI-Bezeichnungen Caprylyl/Capryl Glucoside, Decyl Glucoside, Lauryl Glucoside und/oder Coco Glucoside enthalten. Insbesondere bevorzugt sind Decyl Glucoside und/oder Coco Glucoside enthalten.

Vorteilhaft ist es ebenfalls, wenn der Anteil der Alkyl(oligo)glycosid der allgemeinen Formel RO-[G]ₓ, insbesondere der Anteil von Decyl Glucoside und/oder Coco Glucoside, von 1,5 bis 5 Gew.-%, bevorzugt von 2 bis 4 Gew.-% und insbesondere bevorzugt von 2,3 bis 3,3 Gew.-% beträgt, jeweils bezogen auf das Gesamtgewicht der kosmetischen Reinigungszubereitung.

Vorteilhafte kosmetische Reinigungszubereitungen der Erfindung sind außerdem dadurch gekennzeichnet, dass diese PEG-40 Hydrogenated Castor Oil enthalten. Enthält die Reinigungszubereitung PEG-40 Hydrogenated Castor Oil, so beträgt der Anteil von PEG-40 Hydrogenated Castor Oil vorteilhaft von 0,2 bis 1 Gew.-%, insbesondere 0,4 bis 0,85 Gew.-%, bezogen auf das Gesamtgewicht der kosmetischen Reinigungszubereitung.

Ferner ist es vorteilhaft, wenn die kosmetische Reinigungszubereitung Zitronensäure in einem Anteil von 0,3 bis 0,8 Gew.-% bezogen auf das Gesamtgewicht der Zubereitung, enthält.

Vorteilhaft ist es ferner, wenn zusätzlich Natriumbenzoat in Anteilen von 0,4 bis 0,6 Gew.-%, bezogen auf das Gesamtgewicht der kosmetischen Reinigungszubereitung, enthalten ist.

Zusätzlich ist es vorteilhaft, wenn die kosmetische Reinigungszubereitung dadurch gekennzeichnet ist, dass Guar Hydroxypropyltrimonium Chlorid enthalten ist, wobei der Anteil von Guar Hydroxypropyltrimonium Chlorid bevorzugt von 0,1 bis 0,3 Gew.-% bezogen auf das Gesamtgewicht der kosmetischen Reinigungszubereitung beträgt.

Die Viskosität der erfindungsgemäßen Reinigungszubereitung beträgt vorteilhaft bei 25°C 3000 bis 8000 mPa s, wobei insbesondere vorteilhafte Ergebnisse bei einer Viskosität von 3500 bis 5000 mPa s erzielt werden. Die angegebenen Viskositätswerte beziehen sich auf Messungen, wie vorstehend beschrieben.

Ferner weisen die erfindungsgemäßen Reinigungszubereitungen vorteilhaft einen pH-Wert im Bereich von 4,0 bis 4,8 und insbesondere im Bereich von 4,2 bis 4,6 auf.

Der pH-Wert kann erfindungsgemäß durch Zugabe von Zitronensäure und/oder Natronlauge eingestellt werden.

Weiterhin sind die erfindungsgemäßen Reinigungszubereitungen dadurch gekennzeichnet, dass diese kein Natriumlaurylethersulfat (Sodium Laureth Sulfate) enthalten, so dass die Zubereitung zu Anwendung auf sensibler Kinderhaut insbesondere geeignet sind.

Ferner sind die Reinigungszubereitungen der Erfindung dadurch gekennzeichnet, dass diese keine Polymere enthalten sind, welche aus Homo- oder Copolymerisation mit Acrylsäure und/oder Methacrylsäure erhalten werden, und keine Polymere enthalten sind, welche gemäß INCI-Bezeichnung mit Polyquaternium-Z bezeichnet werden, wobei Z eine ganzzahlige Zahl im Bereich von 1 bis 100 ist. Auf diese Weise können Reinigungszubereitungen bereitgestellt werden, welche den ökologischen Anforderungen entsprechen.

Ferner sind erfindungsgemäß vorteilhafte Reinigungszubereitungen dadurch gekennzeichnet, dass diese Wasser in einem Anteil von 60 Gew.-% bis 95 Gew.-% und bevorzugt von 70 Gew.-% bis 90 Gew.-%, bezogen auf das Gesamtgewicht der Reinigungszubereitung enthalten.

Ferner können die erfindungsgemäßen Reinigungszubereitungen weitere Inhaltstoffe, wie Farbstoffe; Pigmente; Partikel aus mikrokristalliner Cellulose, Silicat oder Hydrogenated Castor Öl; Wirkstoffe; flüssige Parfümstoffe und/oder natürliche Öle enthalten.

### Vergleichsversuche und Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **Inhaltsstoffe** | **Bsp. 1** | **Bsp. 2** | **Bsp. 3** | **Bsp. 4** | **Bsp. 5** | **Bsp. 6** |
|---|---|---|---|---|---|---|
| Sodium Myreth Sulfate | 4,81 | 5,15 | 4,97 | 4,56 | 5,34 | 4,75 |
| PEG-200 Hydrogenated Glyceryl Palmate | 0,30 | 0,25 | 0,35 | 0,20 | 0,40 | 0,45 |
| PEG-90 Glyceryl Isostearate | 0,30 | 0,25 | 0,35 | 0,20 | 0,40 | 0,45 |
| Denatonium Benzoate | 0,001 | 0,004 | 0,003 | 0,002 | 0,005 | 0,002 |
| Guar Hydroxypropyltrimonium Chloride | 0,2 | | 0,18 | | | 0,15 |
| Cocamidopropyl Betaine | 4,53 | | 5,56 | 4,38 | | 5,26 |
| Lauramidopropyl Betaine | | 5,15 | | | 6,12 | |
| Decyl Glucoside | 3,15 | 2,70 | 2,65 | 2,55 | 3,00 | |
| Coco-Glucoside | | | | | | 2,90 |
| Glycol Distearate | 0,55 | | 0,60 | 0,65 | | 0,75 |
| PEG-3 Distearate | | 0,70 | | | 0,55 | |
| Sodium Benzoate | 0,40 | 0,35 | 0,45 | 0,45 | 0,40 | 0,45 |
| PEG-40 Hydrogenated Castor Oil | 0,50 | 0,30 | 1,00 | 1,10 | 0,75 | 0,65 |
| Citric Acid | 0,50 | 0,75 | 0,63 | 0,46 | 0,38 | 0,55 |
| Sodium Hydroxide | 0,0025 | 0,0061 | 0,0056 | 0,0045 | 0,0036 | 0,0042 |
| Perfume | 0,80 | 1,00 | 0,90 | 0,95 | 1,10 | 0,85 |
| Aqua | ad 100 | ad 100 | ad 100 | ad 100 | ad 100 | ad 100 |

| **Inhaltsstoffe** | **Bsp. 7** | **Bsp. 8** | **Bsp. 9** | **Bsp. 10** | **Bsp. 11** | **Bsp. 12** |
|---|---|---|---|---|---|---|
| Sodium Myreth Sulfate | 5,00 | 5,25 | 4,85 | 5,65 | 4,95 | 5,85 |
| PEG-200 Hydrogenated Glyceryl Palmate | 0,50 | 0,15 | 0,75 | 0,10 | 0,60 | 1,00 |
| PEG-90 Glyceryl Isostearate | 0,50 | 0,15 | 0,75 | 0,10 | 0,60 | 1,00 |
| Denatonium Benzoate | 0,003 | 0,005 | 0,002 | 0,004 | 0,001 | 0,003 |
| Guar Hydroxypropyltrimonium Chloride | | 0,25 | | 0,38 | 0,10 | |
| Cocamidopropyl Betaine | | 5,75 | 4,88 | | 6,05 | |
| Lauramidopropyl Betaine | 4,95 | | | 5,15 | | 4,85 |
| Decyl Glucoside | | 2,85 | 2,95 | | | 2,65 |
| Coco-Glucoside | 3,05 | | | 2,45 | 3,10 | |
| Glycol Distearate | | 0,80 | 0,70 | | 0,85 | |
| PEG-3 Distearate | 0,65 | | | 0,45 | | 0,60 |
| Sodium Benzoate | 0,40 | 0,50 | 0,40 | 0,45 | 0,50 | 0,35 |
| PEG-40 Hydrogenated Castor Oil | 0,25 | 0,60 | 0,90 | 0,85 | 0,65 | 0,80 |
| Citric Acid | 0,50 | 0,75 | 0,62 | 0,46 | 0,38 | 0,55 |
| Sodium Hydroxide | 0,0012 | 0,0058 | 0,0063 | 0,0037 | 0,0049 | 0,0065 |
| Perfume | 0,85 | 0,90 | 1,00 | 0,80 | 0,95 | 1,1 |
| Aqua | ad 100 | ad 100 | ad 100 | ad 100 | ad 100 | ad 100 |

In einem Vergleichsversuch wurden Beispiel 3 einmal eingesetzt, wie in der Tabelle beschrieben. Zudem wurde die Rezeptur einmal ohne Denatonium Benzoate hergestellt. Beide Rezepturen wurden nach Herstellung 2 Monate bei 40°C gelagert. Nach Lagerung konnten in der Rezeptur ohne Denatonium Benzoate Ablagerungen von Perlglanzpartikeln am Boden erkannt werden. Das Beispiel 3 zeigte hingegen keine Ablagerung von Partikeln und war somit homogen und stabil.

## Patentansprüche

1. Kosmetische Reinigungszubereitung umfassend
a) Sodium Myreth Sulfate;
b) mindestens ein amphoteres Tensid enthaltend eine Betainegruppe;
c) Denatonium Benzoate; und
d) mindestens einen Perlglanzpartikel gewählt aus der Gruppe PEG-3 Distearate und Glycol Distearate
**dadurch gekennzeichnet, dass** die kosmetische Reinigungszubereitung
• kein Natriumlaurylethersulfat enthält und
• keine Polymere enthalten sind, welche aus Homo- oder Copolymerisation mit Acrylsäure und/oder Methacrylsäure erhalten werden, und/oder keine Polymere enthalten sind, welche gemäß INCI-Bezeichnung mit Polyquaternium-Z bezeichnet werden, wobei Z eine ganzzahlige Zahl im Bereich von 1 bis 100 ist.

2. Verwendung einer Kombination aus Sodium Myreth Sulfate, mindestens ein amphoteres Tensid enthaltend eine Betainegruppe und Denatonium Benzoate in einer kosmetischen Reinigungszubereitung enthaltend mindestens einen Perlglanzpartikel gewählt aus der Gruppe PEG-3 Distearate und Glycol Distearate zur Stabilisierung der Perlglanzpartikel in der Reinigungszubereitung, wobei die kosmetische Reinigungszubereitung kein Natriumlaurylethersulfat enthält.

3. Reinigungszubereitung oder Verwendung nach einem der vorhergehenden Ansprüche **dadurch gekennzeichnet, dass** der Anteil von Sodium Myreth Sulfate von 4 bis 8 Gew.-%, bevorzugt von 4,2 bis 6 Gew.-% und insbesondere bevorzugt von 4,5 bis 5,5 Gew.-%, jeweils bezogen auf das Gesamtgewicht der Reinigungszubereitung beträgt.

4. Reinigungszubereitung oder Verwendung nach einem der vorhergehenden Ansprüche **dadurch gekennzeichnet, dass** der Gesamtanteil aller enthaltenen anionischen Tenside kleiner als 8,5 Gew.-%, bevorzugt kleiner 6,5 Gew.-% und insbesondere bevorzugt kleiner 5,6 Gew.-% beträgt, wobei sich die Angaben auf das Gesamtgewicht der Reinigungszubereitung beziehen.

5. Reinigungszubereitung oder Verwendung nach einem der vorhergehenden Ansprüche **dadurch gekennzeichnet, dass** als amphoteres Tensid enthaltend eine Betainstruktur Cocamidopropylbetaine und/oder Lauramidopropylbetaine enthalten sind.

6. Reinigungszubereitung oder Verwendung nach einem der vorhergehenden Ansprüche **dadurch gekennzeichnet, dass** der Gesamtanteil der amphoteren Tenside enthaltend eine Betainstruktur von 3 bis 8 Gew.-%, bevorzugt von 4 bis 7 Gew.-% und insbesondere bevorzugt von 4,5 bis 6,5 Gew.-% bezogen auf das Gesamtgewicht der kosmetischen Reinigungszubereitung beträgt.

7. Reinigungszubereitung oder Verwendung nach einem der vorhergehenden Ansprüche **dadurch gekennzeichnet, dass** das Gewichtsverhältnis von den anionischen Tensiden zu den amphoteren Tensiden in der Reinigungszubereitung von 1,5:1 bis 1:1,5, bevorzugt von 1,2:1 bis 1:1,2 und insbesondere bevorzugt von 1,1:1 bis 1:1,1 beträgt.

8. Reinigungszubereitung oder Verwendung nach einem der vorhergehenden Ansprüche **dadurch gekennzeichnet, dass** der Anteil von Tensiden, welche eine anionische und/oder kationische Ladung aufweisen, weniger als 12 Gew.-%, bevorzugt weniger als 11 Gew.-% und insbesondere bevorzugt weniger als 10,5 Gew.-% bezogen auf das Gesamtgewicht der Zubereitung beträgt.

9. Reinigungszubereitung oder Verwendung nach einem der vorhergehenden Ansprüche **dadurch gekennzeichnet, dass** Denatonium Benzoate in einem Anteil von 0,001 bis 0,01 Gew.-%, bevorzugt 0,002 bis 0,008 Gew.-% und insbesondere bevorzugt von 0,0025 bis 0,005 Gew.-% enthalten ist, wobei sich die Angaben auf das Gesamtgewicht der Zubereitung beziehen.

10. Reinigungszubereitung oder Verwendung nach einem der vorhergehenden Ansprüche **dadurch gekennzeichnet, dass** der Anteil der Perlglanzpartikel gewählt aus der Gruppe PEG-3 Distearate und Glycol Distearate von 0,1 bis 1,5 Gew.-%, bevorzugt 0,2 bis 1 Gew.-% und insbesondere bevorzugt 0,3 bis 0,8 Gew.-% beträgt, jeweils bezogen auf das Gesamtgewicht der kosmetischen Reinigungszubereitung.

11. Reinigungszubereitung oder Verwendung nach einem der vorhergehenden Ansprüche **dadurch gekennzeichnet, dass** PEG-90 Glyceryl Isostearate und/oder PEG-200 Hydrogenated Glyceryl Palmate in der Reinigungszubereitung enthalten sind, wobei es vorteilhaft ist, wenn der Gesamtanteil dieser Substanzen von 0,1 bis 4 Gew.-%, bevorzugt 0,3 bis 2 Gew.-% und insbesondere bevorzugt von 0,4 bis 1,5 Gew.-% beträgt, jeweils bezogen auf das Gesamtgewicht der kosmetischen Reinigungszubereitung.

12. Reinigungszubereitung oder Verwendung nach Anspruch 11 **dadurch gekennzeichnet, dass** der Gewichtsanteil von PEG-90 Glyceryl Isostearate zu PEG-200 Hydrogenated Glyceryl Palmate von 1,4:1 bis 1:1,4, insbesondere bevorzugt von 1,2:1 bis 1:1,2 beträgt.

13. Reinigungszubereitung oder Verwendung nach einem der vorhergehenden Ansprüche **dadurch gekennzeichnet, dass** mindestens ein Alkyl(oligo)glycosid der allgemeinen Formel RO-[G]ₓ in der Reinigungszubereitung enthalten ist, wobei R für einen Alkyl- und/oder Alkylenrest mit 4 bis 22 Kohlenstoffatomen, G für einen Zuckerrest mit 5 oder 6 Kohlenstoffatomen und X für eine ganzzahlige Zahl im Bereich von 1 bis 10 steht.

14. Reinigungszubereitung oder Verwendung nach Anspruch 13 **dadurch gekennzeichnet, dass** der Anteil der Alkyl(oligo)glycosid der allgemeinen Formel RO-[G]ₓ, insbesondere der Anteil von Decyl Glucoside und/oder Coco Glucoside, von 1,5 bis 5 Gew.-%, bevorzugt von 2 bis 4 Gew.-% und insbesondere bevorzugt von 2,3 bis 3,3 Gew.-% beträgt, jeweils bezogen auf das Gesamtgewicht der kosmetischen Reinigungszubereitung.

15. Verwendung nach einem der vorhergehenden Ansprüche **dadurch gekennzeichnet, dass** keine Polymere enthalten sind, welche aus Homo- oder Copolymerisation mit Acrylsäure und/oder Methacrylsäure erhalten werden, und/oder keine Polymere enthalten sind, welche gemäß INCI-Bezeichnung mit Polyquaternium-Z bezeichnet werden, wobei Z eine ganzzahlige Zahl im Bereich von 1 bis 100 ist.

## Claims

1. Cosmetic cleansing preparation comprising
a) sodium myreth sulfate;
b) at least one amphoteric surfactant comprising a betaine group;
c) denatonium benzoate; and
d) at least one pearlescent particle selected from the group of PEG-3 distearate and glycol distearate
**characterized in that** the cosmetic cleansing preparation
• contains no sodium lauryl ether sulfate and
• no polymers are present obtained from homo- or copolymerization with acrylic acid and/or methacrylic acid, and/or no polymers are present which are designated in accordance with the INCI name as polyquaternium-Z, where Z is an integer in the range of 1 to 100.

2. Use of a combination of sodium myreth sulfate, at least one amphoteric surfactant comprising a betaine group and denatonium benzoate in a cosmetic cleansing preparation comprising at least one pearlescent particle selected from the group of PEG-3 distearate and glycol distearate for stabilizing the pearlescent particles in the cleansing preparation, wherein the cosmetic cleansing preparation does not comprise any sodium lauryl ether sulfate.

3. Cleansing preparation or use according to either of the preceding claims, **characterized in that** the proportion of sodium myreth sulfate is from 4 to 8% by weight, preferably from 4.2 to 6% by weight and particularly preferably from 4.5 to 5.5% by weight, based in each case on the total weight of the cleansing preparation.

4. Cleansing preparation or use according to any of the preceding claims, **characterized in that** the total proportion of all anionic surfactants present is less than 8.5% by weight, preferably less than 6.5% by weight and particularly preferably less than 5.6% by weight, wherein the figures refer to the total weight of the cleansing preparation.

5. Cleansing preparation or use according to any of the preceding claims, **characterized in that** cocamidopropyl betaine and/or lauramidopropyl betaine are present as amphoteric surfactant comprising a betaine structure.

6. Cleansing preparation or use according to any of the preceding claims, **characterized in that** the total proportion of amphoteric surfactants comprising a betaine structure is from 3 to 8% by weight, preferably from 4 to 7% by weight and particularly preferably from 4.5 to 6.5% by weight, based on the total weight of the cosmetic cleansing preparation.

7. Cleansing preparation or use according to any of the preceding claims, **characterized in that** the ratio by weight of anionic surfactants to amphoteric surfactants in the cleansing preparation is from 1.5:1 to 1:1.5, preferably from 1.2:1 to 1:1.2 and particularly preferably from 1.1:1 to 1:1.1.

8. Cleansing preparation or use according to any of the preceding claims, **characterized in that** the proportion of surfactants having an anionic and/or cationic charge is less than 12% by weight, preferably less than 11% by weight and particularly preferably less than 10.5% by weight, based on the total weight of the preparation.

9. Cleansing preparation or use according to any of the preceding claims, **characterized in that** denatonium benzoate is present at a proportion of 0.001 to 0.01% by weight, preferably 0.002 to 0.008% by weight and particularly preferably from 0.0025 to 0.005% by weight, wherein the figures refer to the total weight of the preparation.

10. Cleansing preparation or use according to any of the preceding claims, **characterized in that** the proportion of pearlescent particles selected from the group of PEG-3 distearate and glycol distearate is from 0.1 to 1.5% by weight, preferably 0.2 to 1% by weight and particularly preferably 0.3 to 0.8% by weight, based in each case on the total weight of the cosmetic cleansing preparation.

11. Cleansing preparation or use according to any of the preceding claims, **characterized in that** PEG-90 glyceryl isostearate and/or PEG-200 hydrogenated glyceryl palmate are present in the cleansing preparation, wherein it is advantageous when the total proportion of said substances is from 0.1 to 4% by weight, preferably 0.3 to 2% by weight and particularly preferably from 0.4 to 1.5% by weight, based in each case on the total weight of the cosmetic cleansing preparation.

12. Cleansing preparation or use according to Claim 11, **characterized in that** the proportion by weight of PEG-90 glyceryl isostearate to PEG-200 hydrogenated glyceryl palmate is from 1.4:1 to 1:1.4, particularly preferably from 1.2:1 to 1:1.2.

13. Cleansing preparation or use according to any of the preceding claims, **characterized in that** at least one alkyl (oligo)glycoside of the general formula RO-[G]ₓ is present in the cleansing preparation, where R is an alkyl and/or alkylene radical having 4 to 22 carbon atoms, G is a sugar radical having 5 or 6 carbon atoms and X is an integer in the range of 1 to 10.

14. Cleansing preparation or use according to Claim 13, **characterized in that** the proportion of the alkyl (oligo)glycoside of the general formula RO-[G]ₓ, in particular the proportion of decyl glucoside and/or coco glucoside, is from 1.5 to 5% by weight, preferably from 2 to 4% by weight and particularly preferably from 2.3 to 3.3% by weight, based in each case on the total weight of the cosmetic cleansing preparation.

15. Use according to any of the preceding claims, **characterized in that** no polymers are present obtained from homo- or copolymerization with acrylic acid and/or methacrylic acid, and/or no polymers are present which are designated in accordance with the INCI name as polyquaternium-Z, where Z is an integer in the range of 1 to 100.

## Revendications

1. Préparation de nettoyage cosmétique comprenant
a) du Sodium Myreth Sulfate ;
b) au moins un tensioactif amphotère contenant un groupe bétaïne ;
c) du benzoate de dénatonium ; et
d) au moins une particule nacrée choisie dans le groupe du PEG-3 Distearate et du Glycol Distearate
**caractérisée en ce que** la préparation de nettoyage cosmétique
• ne contient pas de lauryléthersulfate de sodium et
• ne contient pas de polymères qui sont obtenus par homopolymérisation ou copolymérisation avec l'acide acrylique et/ou l'acide méthacrylique, et/ou ne contient pas de polymères qui sont dénommés Polyquaternium-Z selon la dénomination INCI, Z étant un nombre entier dans la plage de 1 à 100.

2. Utilisation d'une combinaison de Sodium Myreth Sulfate, d'au moins un tensioactif amphotère contenant un groupe bétaïne et de benzoate de dénatonium dans une préparation de nettoyage cosmétique contenant au moins une particule nacrée choisie dans le groupe du PEG-3 Distearate et du Glycol Distearate pour la stabilisation des particules nacrées dans la préparation de nettoyage, la préparation de nettoyage cosmétique ne contenant pas de lauryléthersulfate de sodium.

3. Préparation de nettoyage ou utilisation selon l'une quelconque des revendications précédentes, **caractérisée en ce que** la proportion de Sodium Myreth Sulfate est de 4 à 8 % en poids, préférablement de 4,2 à 6 % en poids et particulièrement préférablement de 4,5 à 5,5 % en poids, à chaque fois par rapport au poids total de la préparation de nettoyage.

4. Préparation de nettoyage ou utilisation selon l'une quelconque des revendications précédentes, **caractérisée en ce que** la proportion totale de tous les tensioactifs anioniques contenus est inférieure à 8,5 % en poids, préférablement inférieure à 6,5 % en poids et particulièrement préférablement inférieure à 5,6 % en poids, les données se rapportant au poids total de la préparation de nettoyage.

5. Préparation de nettoyage ou utilisation selon l'une quelconque des revendications précédentes, **caractérisée en ce que** la cocamidopropylbétaïne et/ou la lauramidopropylbétaïne sont contenues en tant que tensioactif amphotère contenant une structure bétaïne.

6. Préparation de nettoyage ou utilisation selon l'une quelconque des revendications précédentes, **caractérisée en ce que** la proportion totale des tensioactifs amphotères contenant une structure bétaïne est de 3 à 8 % en poids, préférablement de 4 à 7 % en poids et particulièrement préférablement de 4,5 à 6,5 % en poids, par rapport au poids total de la préparation de nettoyage.

7. Préparation de nettoyage ou utilisation selon l'une quelconque des revendications précédentes, **caractérisée en ce que** le rapport en poids des tensioactifs anioniques sur les tensioactifs amphotères dans la préparation de nettoyage est de 1,5 : 1 à 1 : 1,5, préférablement de 1,2 : 1 à 1 : 1,2 et particulièrement préférablement de 1,1 : 1 à 1 : 1,1.

8. Préparation de nettoyage ou utilisation selon l'une quelconque des revendications précédentes, **caractérisée en ce que** la proportion de tensioactifs qui présentent une charge anionique et/ou cationique est inférieure à 12 % en poids, préférablement inférieure à 11 % en poids et particulièrement préférablement inférieure à 10,5 % en poids par rapport au poids total de la préparation.

9. Préparation de nettoyage ou utilisation selon l'une quelconque des revendications précédentes **caractérisée en ce que** le benzoate de dénatonium est contenu en une proportion de 0,001 à 0,01 % en poids, préférablement de 0,002 à 0,008 % en poids et particulièrement préférablement de 0,0025 à 0,005 % en poids, les données se rapportant au poids total de la préparation.

10. Préparation de nettoyage ou utilisation selon l'une quelconque des revendications précédentes **caractérisée en ce que** la proportion des particules nacrées choisies dans le groupe du PEG-3 Distearate et du Glycol Distearate est de 0,1 à 1,5 % en poids, préférablement de 0,2 à 1 % en poids et particulièrement préférablement de 0,3 à 0,8 % en poids, à chaque fois par rapport au poids total de la préparation de nettoyage cosmétique.

11. Préparation de nettoyage ou utilisation selon l'une quelconque des revendications précédentes, **caractérisée en ce que** du PEG-90 Glyceryl Isostearate et/ou du PEG-200 Hydrogenated Glyceryl Palmate est/sont contenu(s) dans la préparation de nettoyage, dans laquelle il est avantageux que la proportion globale de ces substances soit de 0,1 à 4 % en poids, préférablement 0,3 à 2 % en poids et particulièrement préférablement de 0,4 à 1,5 % en poids, à chaque fois par rapport au poids total de la préparation de nettoyage cosmétique.

12. Préparation de nettoyage ou utilisation selon la revendication 11, **caractérisée en ce que** la proportion en poids de PEG-90 Glyceryl Isostearate à PEG-200 Hydrogenated Glyceryl Palmate est de 1,4 : 1 à 1 : 1,4, particulièrement préférablement de 1,2 : 1 à 1 : 1,2.

13. Préparation de nettoyage ou utilisation selon l'une quelconque des revendications précédentes, **caractérisée en ce qu'**au moins un alkyl(oligo)glycoside de formule générale RO-[G]ₓ est contenu dans la préparation de nettoyage, R représentant un radical alkyle et/ou alkylène comportant 4 à 22 atomes de carbone, G représentant un radical de sucre comportant 5 ou 6 atomes de carbone et X représentant un nombre entier dans la plage de 1 à 10.

14. Préparation de nettoyage ou utilisation selon la revendication 13, **caractérisée en ce que** la proportion d'alkyl(oligo)glycoside de formule générale RO-[G]ₓ, en particulier la proportion de Decyl Glucoside et/ou de Coco Glucoside, est de 1,5 à 3 % en poids, préférablement de 2 à 4 % en poids et en particulier préférablement de 2,3 à 3,3 % en poids, à chaque fois par rapport au poids total de la préparation de nettoyage cosmétique.

15. Utilisation selon l'une quelconque des revendications précédentes, **caractérisée en ce que** ne sont pas contenus de polymères qui sont obtenus par homopolymérisation ou copolymérisation avec l'acide acrylique et/ou l'acide méthacrylique, et/ou ne sont pas contenus de polymères qui sont dénommés Polyquaternium-Z selon la dénomination INCI, Z étant un nombre entier dans la plage de 1 à 100.
